# EUROPEAN PATENT SPECIFICATION

(11) **EP 2 030 489 B1**
(45) Date of publication and mention of the grant of the patent: **23.11.2016**
(21) Application number: 07777022.0
(22) Date of filing: 14.05.2007
(51) Int. Cl.: H05G 1/00, G21K 5/00, G21K 5/08, A61B 6/04, A47C 31/00, A61G 7/07, A61G 1/04, A61B 6/03, A61B 6/00

(54) **X-RAY TRANSPARENT BED AND GURNEY EXTENDER FOR USE WITH MOBILE COMPUTERIZED TOMOGRAPHY (CT) IMAGING SYSTEMS**
ERWEITERUNG FÜR RÖNTGENSTRAHLENDURCHLÄSSIGES BETT UND KRANKENTRAGE ZUR VERWENDUNG MIT MOBILEN CT-BILDGEBUNGSSYSTEMEN
RALLONGE DE LIT ET DE CIVIÈRE ROULANTE TRANSPARENTE AUX RAYONS X DESTINÉ À ÊTRE UTILISÉS AVEC DES SYSTÈMES MOBILES D'IMAGERIE TOMOGRAPHIQUE PAR ORDINATEUR

(30) Priority: 12.05.2006 US 800107 P; 13.02.2007 US 706133
(43) Date of publication of application: 04.03.2009
(73) Proprietor: Neurologica Corp., Danvers, MA 01923 (US)
(72) Inventor: TYBINKOWSKI, Andrew, P., Boxford, MA 01921 (US); NEMIROVSKY, Lidia, Salem, MA 01970 (US)
(74) Representative: Engelhardt & Engelhardt
(86) International application number: PCT/US2007/011495
(87) International publication number: WO 2007/133723

(56) References cited:
- WO-A1-2005/004723
- WO-A1-2006/013185
- DE-A1- 2 938 261
- DE-A1- 4 406 493
- DE-U1- 29 706 436
- GB-A- 2 405 789
- US-A- 3 631 241
- US-A- 4 064 401
- US-A- 4 506 872
- US-A- 4 688 780
- US-A- 4 971 037
- US-A- 4 989 849
- US-A- 5 233 713
- US-A- 5 422 928
- US-A- 5 675 851
- US-A- 5 771 513
- US-A1- 2002 039 403
- US-A1- 2003 084 512
- US-A1- 2004 158 926
- US-A1- 2005 135 560
- US-A1- 2006 083 355
- US-B1- 6 217 214
- US-B1- 6 295 671
- US-B1- 6 459 923
- US-B2- 6 926 441

## Description

### Field Of The Invention

This invention relates to anatomical imaging systems in general, and more particularly to mobile Computerized Tomography (CT) imaging systems and X-ray transparent bed and gurney extenders for use with the same.

### Background Of The Invention

Strokes are the third leading cause of death in the United States, causing approximately 177,000 deaths per year, and strokes are the number one cause of long-term disability in the United States, currently affecting nearly 5 million people. Strokes are caused by an abrupt interruption of the blood supply to the brain or spinal cord, thereby depriving the tissue of oxygen and resulting in tissue damage.

Strokes typically occur in one of two forms: (i) hemorrhagic stokes, which occur with the rupture of a blood vessel; and (ii) ischemic strokes, which occur with the obstruction of a blood vessel.

Rapid diagnosis is a key component of stroke treatment. This is because the treatment for an ischemic stroke may be contra-indicated for the treatment for a hemorrhagic stroke and, furthermore, the effectiveness of a particular treatment may be time-sensitive. More particularly, the current preferred treatment for an acute ischemic stroke, i.e., the administration of tPA to eliminate clots, is contra-indicated for a hemorrhagic stroke. Furthermore, the clinical data suggests that the medication used to treat ischemic strokes (i.e., tPA) is most effective if it is administered within 3 hours of the onset of the stroke. However, current diagnosis times, i.e., the time needed to identify that the patient is suffering from a stroke and to identify the hemorrhagic or ischemic nature of the stroke, frequently exceeds this 3 hour window. As a result, only a fraction of current ischemic stroke victims are timely treated with tPA.

Imaging is generally necessary to properly diagnose (and hence properly treat) a stroke. More particularly, imaging is generally necessary to: (i) distinguish strokes from other medical conditions; (ii) distinguish between the different types of strokes (i.e., hemorrhagic or ischemic); and (iii) determine appropriate treatments (e.g., the administration of tPA in the case of an ischemic stroke).

Computerized Tomography (CT) has emerged as the key imaging modality in the diagnosis of strokes. CT scanners generally operate by directing X-rays into the body from a variety of positions, detecting the X-rays passing through the body, and then processing the detected X-rays so as to build a computer model of the patient's anatomy. This computer model can then be visualized so as to provide images of the patient's anatomy. It has been found that such CT scanning (including non-enhanced CT scanning, CT angiography scanning and CT perfusion scanning) is able to provide substantially all of the information needed to effectively diagnose (and hence properly treat) a stroke.

Unfortunately, in practice, the CT machine is typically located in the hospital's radiology department and the patient is typically received in the hospital's emergency room, and the "round-trip" time between the emergency room and the radiology department can frequently involve substantial delays, even in the best of hospitals. As a result, the time spent in transporting the patient from the emergency room to the radiology department and then back again can consume critical time which can compromise proper treatment of the patient.

For this reason, as well as others, NeuroLogica Corporation of Danvers, Massachusetts has recently developed a mobile CT imaging system, i.e., the CereTom^{™} CT machine. The CereTom^{™} CT machine is particularly well suited for use in stroke applications. More particularly, the CereTom^{™} CT machine is a small, mobile CT machine which can be pre-positioned in the emergency room and moved to the patient so that the patient can be scanned at their current location, on their emergency room bed or gurney, thus effectively eliminating "round-trip" delays between the emergency room and radiology department and thereby dramatically reducing the time needed to properly diagnose the patient.

The CereTom^{™} CT machine also has application in numerous other situations where patients may be located remote from the CT machine, e.g., other hospital departments such as Intensive Care Units (ICUs), nursing homes, rehabilitation centers, etc.

Since the CereTom^{™} CT machine is designed to be as small and mobile as possible, and since the CereTom^{™} CT machine is intended primarily for stroke applications and thus need only scan the head of the patient, it is configured so as to have a relatively small-diameter scan opening, i.e., a scan opening just large enough to receive the head of the patient. Furthermore, since the beds and gurneys typically found in emergency rooms are too large to fit within the scan opening of the CereTom^{™} CT machine, there is an urgent need for a narrow, X-ray transparent extender for selective attachment to the bed or gurney so as to support the patient's head during scanning, whereby the patient can be quickly and easily scanned while remaining on their bed or

Document US4506872 A, and US3631241 A, disclose a bed or gurney extender according to the preamble of claim 1.

### Summary of the disclosure

These and other objects of the present disclosure are addressed by the provision and use of a narrow, X-ray transparent extender for selective attachment to a bed or gurney so as to support a patient's head during scanning, whereby a patient can be quickly and easily scanned while remaining on their bed or gurney.

In accordance with the present disclosure, there is provided a bed and gurney extender for selective attachment to a standard hospital bed or gurney for supporting the head of a patient during scanning, comprising:
a support for supporting the head of the patient during scanning, wherein at least a portion of the support is X-ray transparent; and
an adapter for selectively attaching the support to the bed or gurney.
Also disclosed is an apparatus for use in scanning a patient on a bed or gurney, comprising:
   a bed and gurney extender for selective attachment to a standard hospital bed or gurney for supporting the head of the patient during scanning, comprising:
      a support for supporting the head of the patient during scanning, wherein at least a portion of the support is X-ray transparent; and
      an adapter for selectively attaching the support to the bed or gurney.

There is also disclosed a method for scanning a patient, comprising:
mounting an extender to the bed or gurney of the patient so as to present the head of the patient on an X-ray transparent support remote from the remainder of the bed or gurney;
positioning the head of the patient adjacent to the scanning zone of a scanner; and
moving the scanner precisely relative to the patient during scanning while the head of the patient remains disposed on the X-ray transparent support.

There is also disclosed a bed and gurney extender for selective attachment to a bed or gurney for supporting the head of a patient during scanning, comprising:
a support for supporting the head of the patient during scanning, wherein at least a portion of the support is transparent to the scanner; and
an adapter for selectively attaching the support to the bed or gurney.

There is also disclosed an apparatus for use in scanning a patient on a bed or gurney, comprising:
a bed and gurney extender for selective attachment to a bed or gurney for supporting the head of the patient during scanning, comprising:
   a support for supporting the head of the patient during scanning, wherein at least a portion of the support is transparent to the scanner; and
   an adapter for selectively attaching the support to the bed or gurney.

There is also disclosed a method for scanning a patient, comprising:
mounting an extender to the bed or gurney of the patient so as to present the head of the patient on a support remote from the remainder of the bed or gurney, wherein the support is transparent to the scanner;
positioning the head of the patient adjacent to the scanning zone of a scanner; and
moving the scanner precisely relative to the patient during scanning while the head of the patient remains disposed on the scanner-transparent support.

### Brief Description Of The Drawings

These and other objects and features of the present disclosure will be more fully disclosed or rendered obvious by the following detailed description of the preferred embodiments of the invention, which is to be considered together with the accompanying drawings wherein like numbers refer to like parts, and further wherein:
Figs. 1 and 2 are schematic external views of a CereTom^{™} CT machine;
Fig. 3 is a schematic internal view of the CereTom^{™} CT machine shown in Figs. 1 and 2;
Fig. 4 is a schematic view showing a bed and gurney extender formed in accordance with the present invention;
Figs. 5 and 6 are schematic views showing the support portion of the bed and gurney extender shown in Fig. 4;
Figs. 7-10 are schematic views showing the adapter portion of the bed and gurney extender shown in Fig. 4;
Figs. 11-14 are schematic views showing how the bed and gurney extender may be stored on a CereTom^{™} CT machine; and
Figs. 15-18 are schematic views showing an alternative form of riser clamp which may be used in connection with the present invention;
Fig. 19 is a schematic view showing an alternative form of the adapter portion of the bed and gurney extender;
Figs. 20 and 21 are schematic views showing still another alternative form of the adapter portion of the bed and gurney extender;
Figs. 22 and 23 are schematic views showing yet another form of the adapter portion of the bed and gurney extender;
Fig. 24 is a schematic view showing another bed and gurney extender formed in accordance with the present invention; and
Fig. 25 is a schematic view showing another support formed in accordance with the present invention.

### Detailed Description Of The Preferred Embodiments

### CereTom^{™} CT Machine

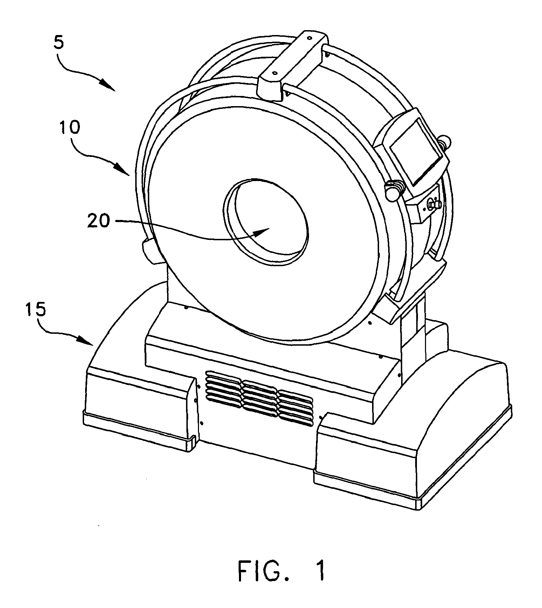

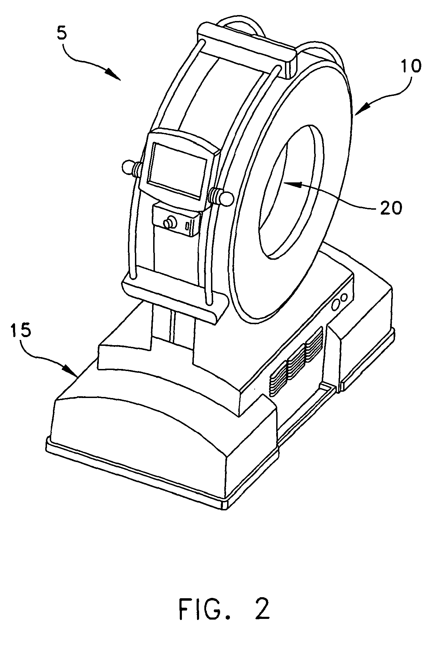

Looking first at Figs. 1 and 2, there is shown a CereTom^{™} CT machine 5. CereTom^{™} CT machine 5 generally comprises a torus 10 which is supported by a base 15. A center opening 20 is formed in torus 10. Center opening 20 receives the patient anatomy which is to be scanned. Since CereTom^{™} CT machine 5 is designed to be as small and mobile as possible, and since CereTom^{™} CT machine 5 is intended primarily for stroke applications and thus need only scan the head of the patient, center opening 20 is configured to be just slightly larger than the head of the patient.

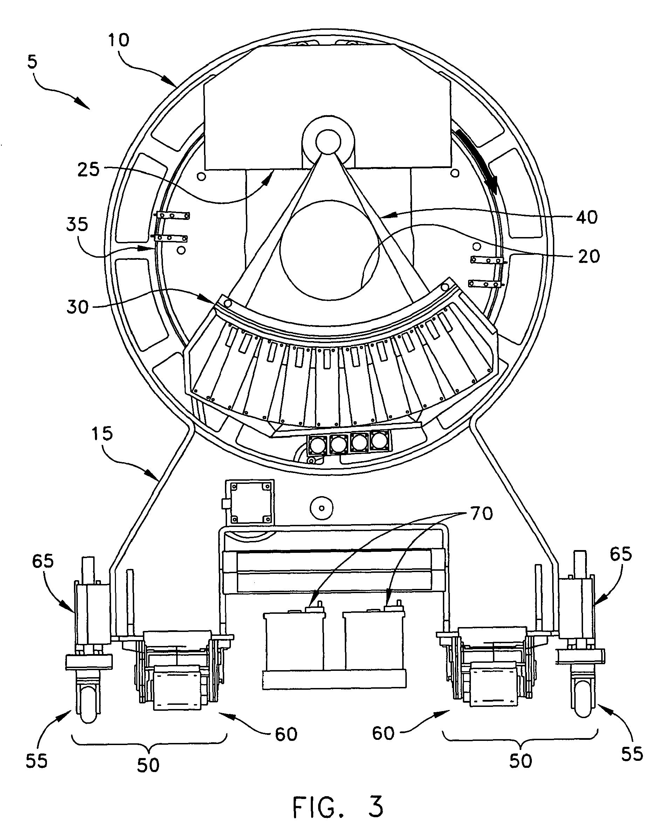

Looking next at Fig. 3, torus 10 generally comprises an X-ray tube assembly 25, an X-ray detector assembly 30, and a rotating drum assembly 35. X-ray tube assembly 25 and X-ray detector assembly 30 are mounted to the rotating drum assembly 35 in diametrically-opposing relation, such that the X-ray beam 40 (generated by X-ray tube assembly 25 and detected by X-ray detector assembly 30) is passed through the patient anatomy disposed in center opening 20. Furthermore, since X-ray tube assembly 25 and X-ray detector assembly 30 are mounted on the rotating drum assembly 35 so that they are rotated concentrically about center opening 20, the X-ray beam 40 will be passed through the patient's anatomy along a full range of radial positions, so as to enable the CereTom^{™} CT machine to create the desired computer model of the scanned anatomy.

The various electronic hardware and software for controlling the operation of X-ray tube assembly 25, X-ray detector assembly 30, and rotating drum assembly 35, as well as for processing the acquired scan data so as to generate the desired computer model, are located in torus 10 and/or base 15.

Still looking now at Fig. 3, base 15 comprises a transport assembly 50 for moving CereTom^{™} CT machine 5 about relative to the patient. More particularly, as disclosed in the aforementioned U.S. Patent Application Serial No. 11/706,133, which patent application is hereby incorporated herein by reference, transport assembly 50 comprises a gross movement mechanism 55 for moving CereTom^{™} CT machine 5 relatively quickly across room distances, and a fine movement mechanism 60 for moving CereTom^{™} CT machine 5 precisely, relative to the patient, during scanning. As discussed in detail in the aforementioned U.S. Patent Application Serial No. 11/706,133, gross movement mechanism 55 preferably comprises a plurality of casters, and fine movement mechanism 60 preferably comprises a plurality of centipede belt drives. Hydraulic apparatus 65 permits either gross movement mechanism 55, or fine movement mechanism 60, to be engaged with the floor, whereby to facilitate appropriate movement of CereTom^{™} CT machine 5.

Base 15 also includes other system components in addition to those discussed above, e.g., batteries 70 for powering various electrical components of CereTom^{™} CT machine 5, etc.

As noted above, the various components of CereTom^{™} CT machine 5 are engineered so as to provide a relatively small, mobile and inexpensive CT machine.

CereTom^{™} CT machine 5 is particularly well suited for use in stroke applications. More particularly, CereTom^{™} CT machine 5 is a small, mobile unit which can be pre-positioned in the emergency room and moved to the patient so that the patient can be scanned at their current location, thus eliminating delays due to patient transport and thereby dramatically reducing the time needed to properly diagnose the patient.

More particularly, the mobile CereTom^{™} CT machine 5 can be located in the emergency room of a hospital and, when a patient presents stroke symptoms, the patient can be immediately scanned in the emergency room so as to determine if the patient is experiencing a stroke and, if so, to determine the nature of the stroke (i.e., hemorrhagic or ischemic). This may be done quickly and easily by moving CereTom^{™} CT machine 5 across the emergency room to the patient's bed or gurney using the casters of gross movement mechanism 55 and then, while the patient remains on their bed or gurney, scanning the patient by precision-advancing CereTom^{™} CT machine 5 relative to the patient using the centipede belt drives of fine movement mechanism 60, so that the scanning zone of CereTom^{™} CT machine 5 is moved relative to the patient. Thus, with CereTom^{™} CT machine 5, the patient can be scanned in the emergency room while remaining on their bed or gurney, without ever having to be moved from the emergency room to the radiology department and then back again, thereby eliminating the traditional scanning delays associated with conventional CT scanners and thus facilitating proper stroke treatment.

As noted above, the CereTom^{™} CT machine 5 also has application in numerous other situations where the patient is located remote from the CT machine, e.g., other hospital departments such as Intensive Care Units (ICUs), nursing homes, rehabilitation centers, etc.

### X-ray Transparent Bed And Gurney Extender

As noted above, CereTom^{™} CT machine 5 is designed to be as small and mobile as possible, and need only scan the head of the patient. As a result, CereTom^{™} CT machine 5 is configured so as to have a relatively small-diameter center scan opening 20 to receive the head of the patient. Since the hospital beds and gurneys typically found in emergency rooms are too large to fit within the scanning area of CereTom^{™} CT machine 5, the present invention provides a narrow, X-ray transparent extender for selective attachment to the bed or gurney so as to support the patient's head during scanning, whereby the patient can be quickly and easily scanned while remaining on their bed or gurney.

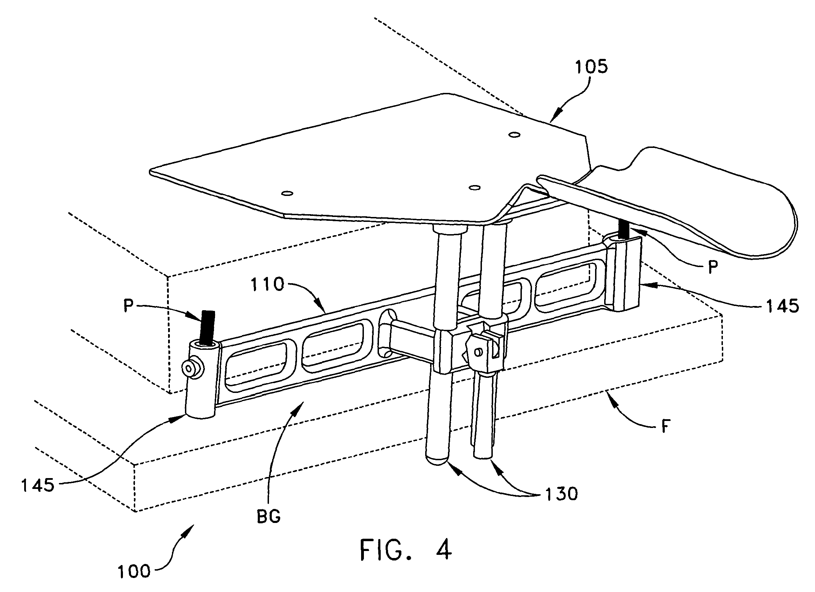

More particularly, and looking now at Fig. 4, there is shown a bed and gurney extender 100 for selective attachment to a bed or gurney BG. For the purposes of the present invention, bed or gurney BG may be a conventional bed or gurney of the type typically found in an emergency room, or it may be any other patient-supporting platform, e.g., an examination table, an operating table, a medical reclining chair, etc.

Extender 100 generally comprises a support 105 for supporting the head of the patient during scanning, and an adapter 110 for selectively attaching support 105 to bed or gurney BG.

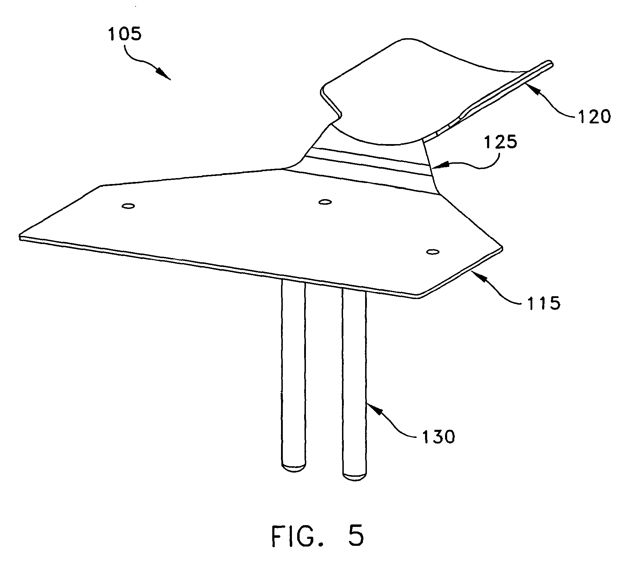

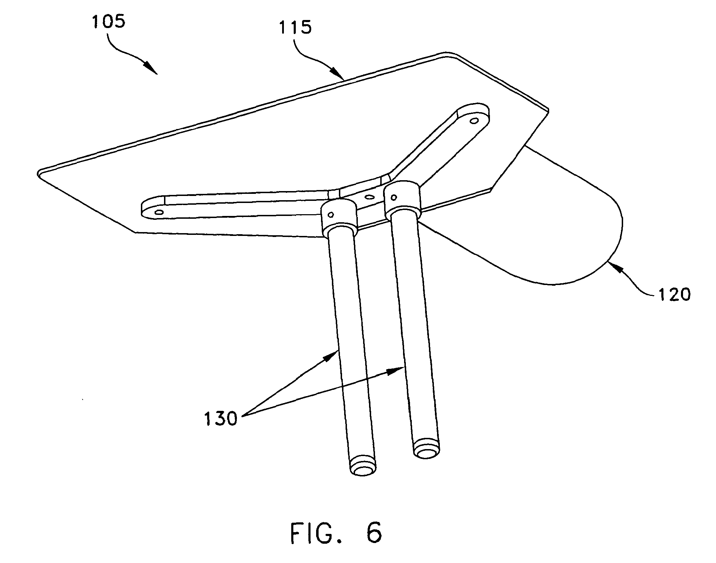

Looking next at Figs. 5 and 6, support 105 generally comprises a shoulder section 115 for disposition on bed or gurney BG, under the shoulders of the patient, and a head section 120 for supporting the head of the patient. Preferably, head section 120 is connected to shoulder section 115 by a neck section 125.

At least head section 120 is formed out of an X-ray transparent material (e.g., carbon fiber, plastic, etc.), such that head section 120 can be in center opening 20 of CereTom^{™} CT machine 5 during scanning. If desired, shoulder section 115 and/or neck section 125 can also be formed out of an X-ray transparent material. At least one riser 130 extends out of the underside of shoulder section 115 for selective attachment to adapter 110. In one preferred form of the invention, two risers 130 are provided.

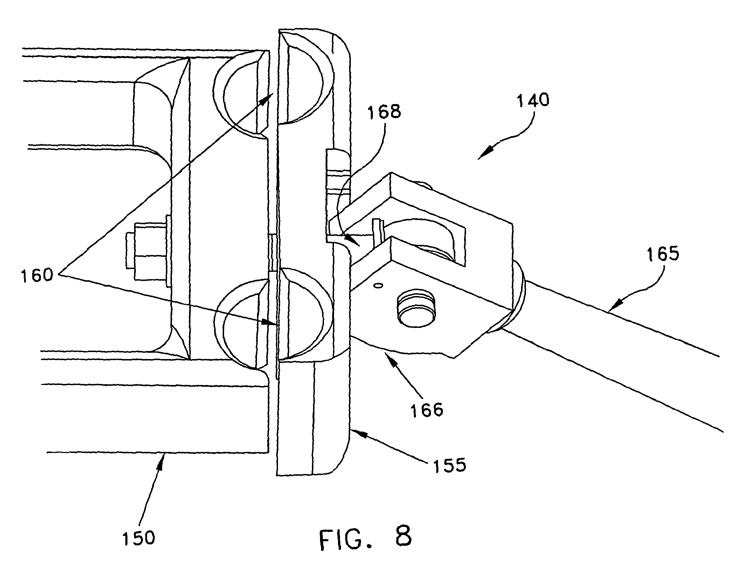

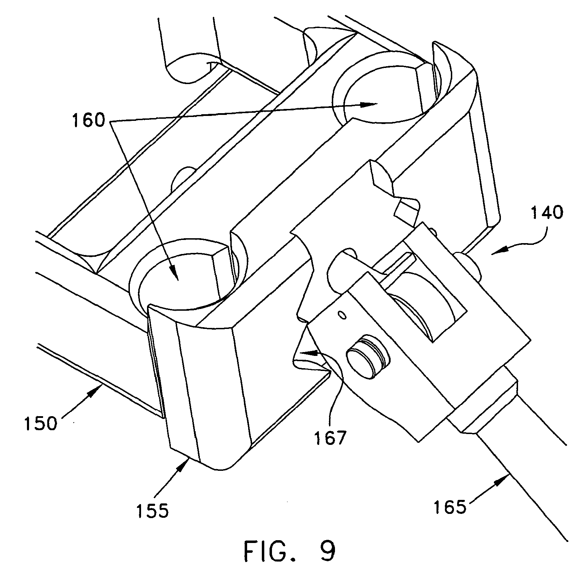

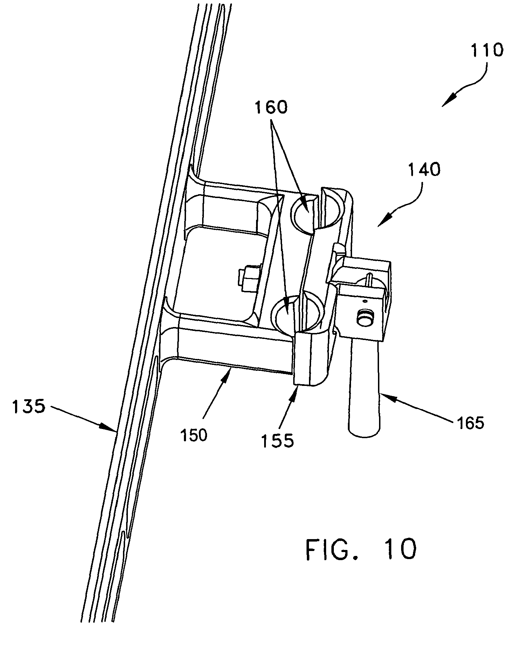

Adapter 110 is shown in Figs. 4 and 7-10. Adapter 110 generally comprises a bed and gurney mount 135 for selective attachment to the bed or gurney BG, and a riser clamp 140 for selectively securing the at least one riser 130 of support 105 to adapter 110.

The specific configuration of bed and gurney mount 135 is chosen according to the specific configuration of bed or gurney BG. Thus, for example, as seen in Fig. 4, where bed or gurney BG comprises a pair of posts P upstanding from the frame F of bed or gurney BG, bed and gurney mount 135 may comprise a pair of receptacles 145 for receiving posts P, whereby to secure bed and gurney mount 135 to the bed or gurney BG.

Of course, other bed and gurney configurations exist in the marketplace, and hence other configurations may be provided for bed and gurney mount 135, with the specific configuration of bed and gurney mount 135 being matched to the particular configuration of the bed or gurney BG with which the extender 100 is to be used.

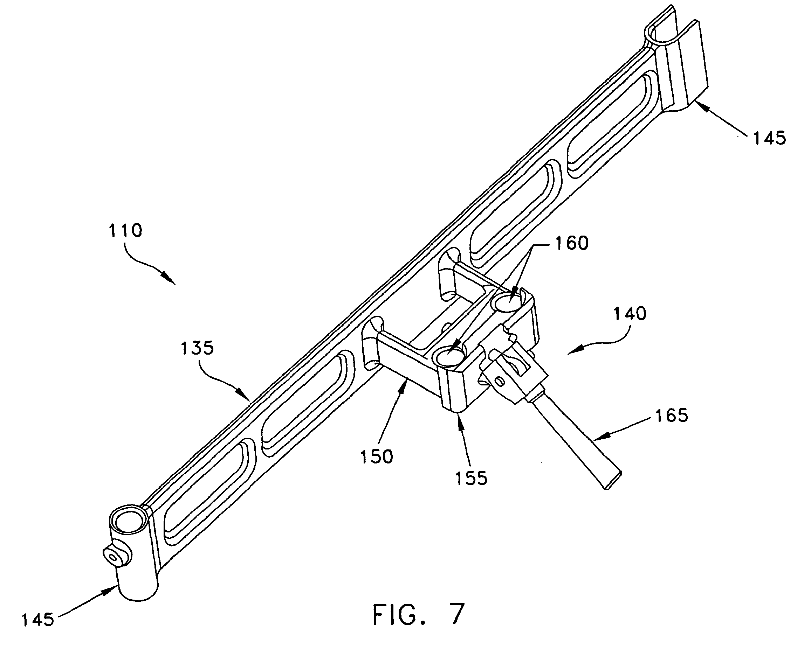

Riser clamp 140 comprises a fixed plate 150 and a movable plate 155 which together define at least one variably-sized opening 160 for selectively receiving and securing the at least one riser 130 of support 105. In one preferred form of the invention, two variably-sized openings 160 are provided for receiving the two risers 130 of support 105. In the construction shown in Figs. 7-10, a handle 165 manipulates a cam mechanism for opening and closing riser clamp 140. In one preferred construction (Figs. 7-10), handle 165 has a cam surface 166 which bears against plate surface 167 so as to selectively position movable plate 155 along shaft 168, whereby to open and close riser clamp 140. Thus it will be seen that when riser clamp 140 is in its open position, risers 130 can be slidably received in openings 160; and when riser clamp 140 is in its closed position, risers 130 are secured to adapter 110, whereby to secure support 105 to bed or gurney BG.

### Use

The apparatus may be used as follows.

When a patient arrives at the emergency room presenting stroke-like symptoms, they are quickly scanned in the emergency room, on their bed or gurney, using CereTom^{™} CT machine 5 (which is pre-positioned in the emergency room) and extender 100. More particularly, CereTom^{™} CT machine 5 is raised on its gross movement mechanism 55, i.e., by actuating hydraulic actuators 65. CereTom^{™} CT machine 5 is then moved on its casters to the patient. Extender 100 is secured to the bed or gurney BG by securing adapter 110 to the bed or gurney BG, opening riser clamp 140 if it is not already open, moving risers 130 through openings 160 of riser clamp 140 until the extender's shoulder section 115 lies flat on the top surface of the bed or gurney BG (the patient may be lifted slightly to facilitate this), and then riser clamp 140 is closed, thereby securing support 105 to bed or gurney BG. Then the patient is moved as necessary so that the head of the patient lies on head section 120. Next, CereTom^{™} CT machine 5 (which is still raised on its casters) is moved relative to the bed or gurney BG so that the center opening 20 of CereTom^{™} CT machine 5 is aligned with the patient. Thereafter, hydraulic apparatus 65 is activated so that CereTom^{™} CT machine 5 is supported on its fine movement mechanism 60 (i.e., the centipede belt drives). Scanning is then commenced, with fine movement mechanism 60 precision-advancing (or precision-retracting, or both precision-advancing and precision-retracting) CereTom^{™} CT machine 5 relative to the patient during scanning, with scanning being achieved while the patient remains on their bed or gurney. As scanning occurs, head section 120 (upon which the patient's head is supported) does not inhibit scanning, inasmuch as head section 120 is formed out of an X-ray transparent material.

### Storing Bed And Gurney Extender 100

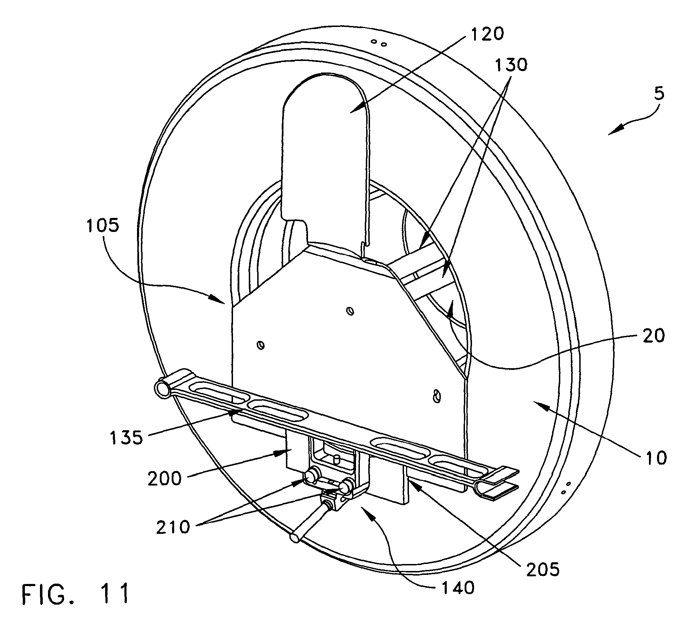

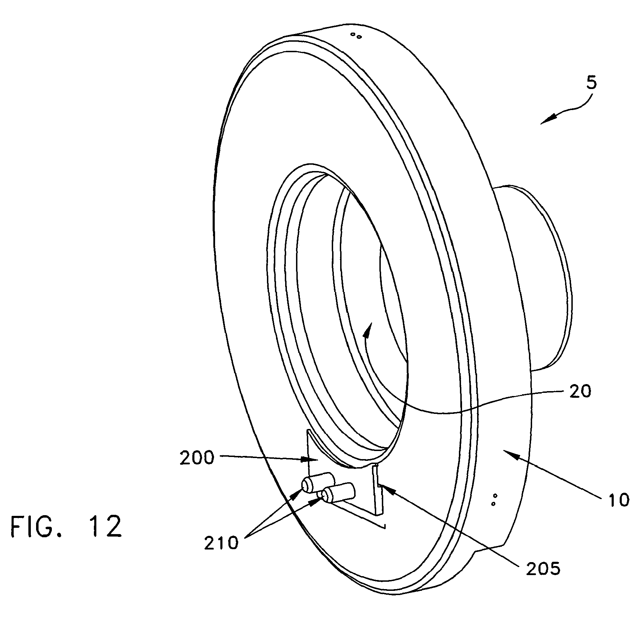

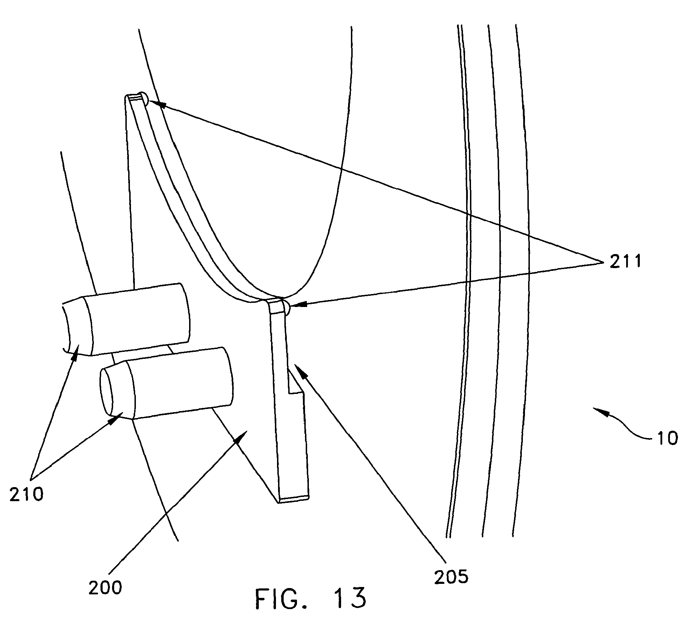

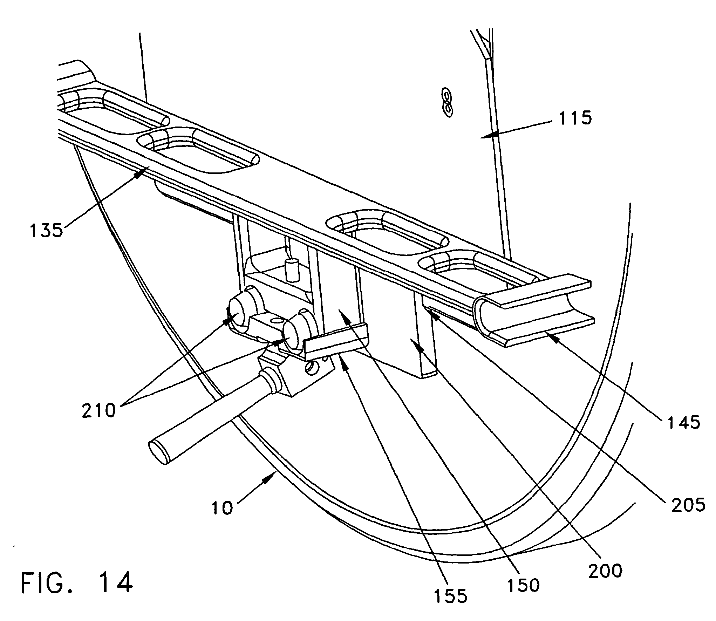

Looking next at Figs. 11-14, CereTom^{™} CT machine 5 can be configured to facilitate storing extender 100 on torus 10 of CereTom^{™} CT machine 5. More particularly, a plate 200, including an undercut 205 and at least one post 210, is attached to the surface of torus 10. Preferably undercut 205 includes two or more beads 211 (Fig. 13) which extend from plate 200 toward CereTom^{™} CT machine 5. Beads 211 may be made of an elastomer, or a plastic, or a metal. When extender 100 is to be stored on CereTom^{™} CT machine 5, risers 130 of support 105 are positioned in center opening 20 of CereTom^{™} CT machine 5 (Fig. 11), and then shoulder section 115 is placed between undercut 205 and torus 10 (Fig. 14), with beads 211 helping to hold shoulder section 115 against torus 10. At the same time, the extender's head section 120 bears against the face of torus 10 (Fig. 11). Then adapter 110 is mounted on the at least one post 210 so as to press shoulder section 115 and head section 120 against torus 10 of CereTom^{™} CT machine 5 (Figs. 11 and 14). This is done by opening riser clamp 140, mounting riser clamp 140 on the at least one post 210, and then closing riser clamp 140 so as to secure adapter 110 and hence extender 100 to CereTom^{™} CT machine 5. Thus it will be seen that the at least one post 210 is preferably arranged so as to have the same configuration as that of the at least one riser 130 (i.e., preferably posts 210 and risers 130 are similar in diameter and, where multiple risers 130 are provided, similar in number and spacing).

### Alternative Riser Clamp Constructions

It should be appreciated that it is possible to provide a riser clamp having a construction which is different from the cam-operated riser clamp shown in Figs. 7-10.

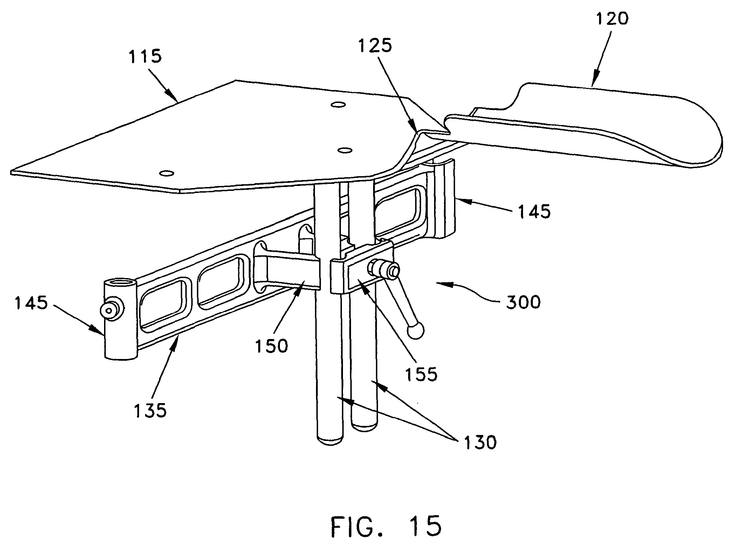

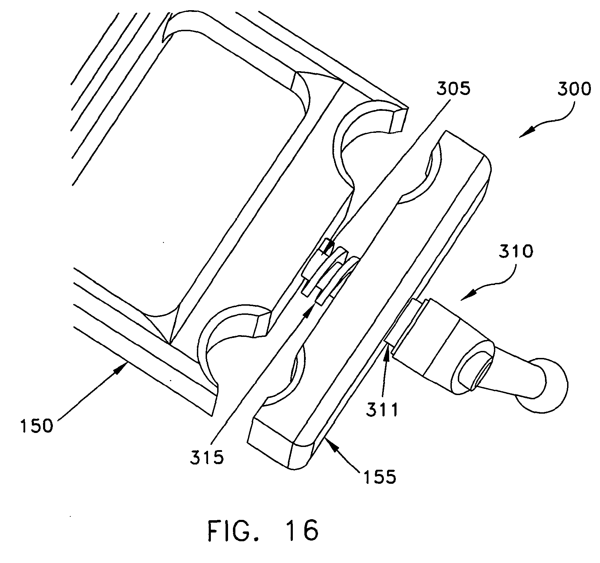

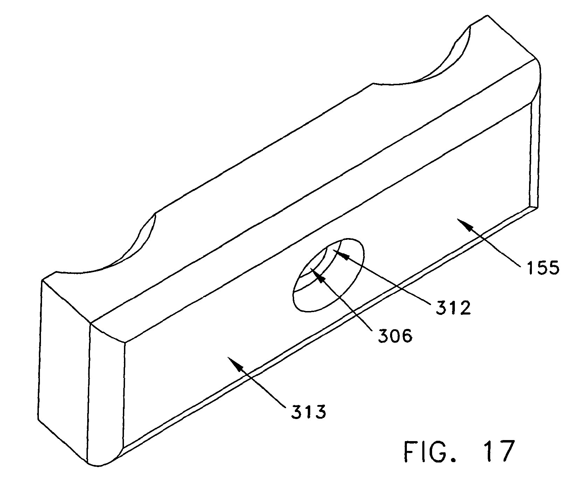

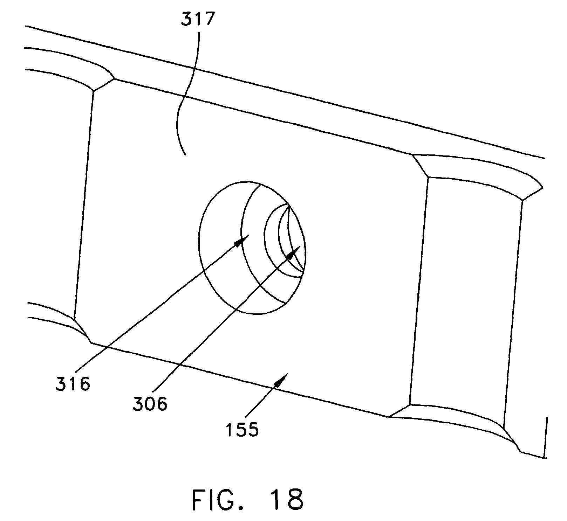

Thus, and looking now at Figs. 15-18, a crank-operated riser clamp 300 is shown. Crank-operated riser clamp 300 generally comprises a screw 305 which has one end mounted in fixed plate 150 and another end passing through a bore 306 formed in movable plate 155. A crank 310 rides on the free end of screw 305. The inner portion 311 of crank 310 bears against an annular shoulder 312 formed in the outside face 313 of movable plate 155. A spring 315 biases movable plate 155 away from fixed plate 150; one end of spring 315 bears against fixed plate 150 and the other end of spring 315 bears against an annular surface 316 formed in the inside face 317 of movable plate 155. As a result of this construction, crank 310 can be used, in conjunction with spring 315, to move movable plate 155 toward and away from fixed plate 150 so as to close and open riser clamp 300.

### Alternative Adapter Constructions

As noted above, many different bed and gurney configurations exist in the marketplace, and hence many different configurations may be provided for bed and gurney mount 135, with the specific configuration of bed and gurney mount 135 being matched to the particular configuration of the bed or gurney BG with which the extender 100 is to be used.

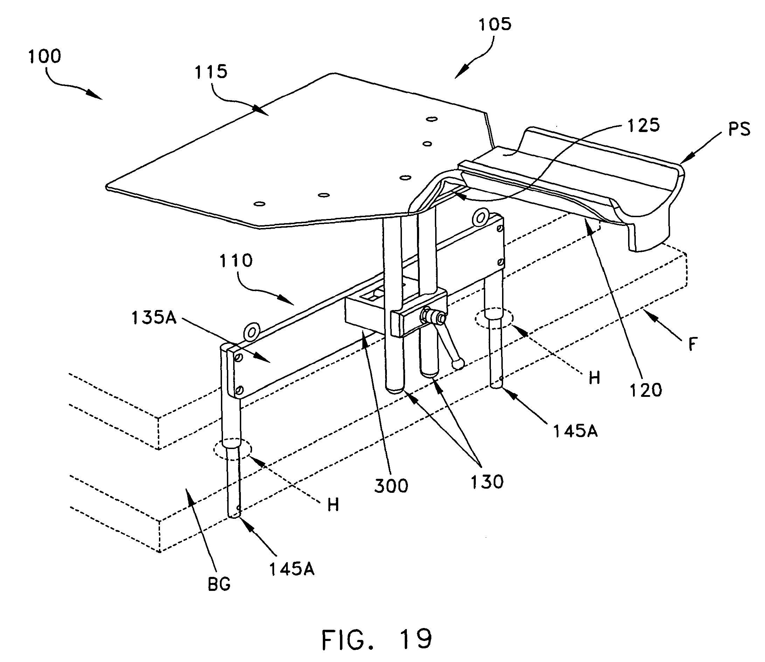

Thus, for example, and looking now at Fig. 19, there is shown an alternative bed and gurney mount 135A. Bed and gurney mount 135A is generally similar to the bed and gurney mount 135 previously discussed, in the sense that the unit mounts to the bed or gurney BG and supports the crank-operated riser clamp 300 (which in turn mounts support 105). However, the alternative bed and gurney mount 135A shown in Fig. 19 is mounted to bed or gurney BG by fitting posts 145A into corresponding holes H formed in the frame F of bed or gurney BG.

If desired, a soft patient support PS may be added to bed and gurney support 105 so as to increase patient comfort. Thus, for example, and looking now at Fig. 19, a patient support PS is shown covering head section 120 and neck section 125. Patient support PS may also cover shoulder section 115 if desired.

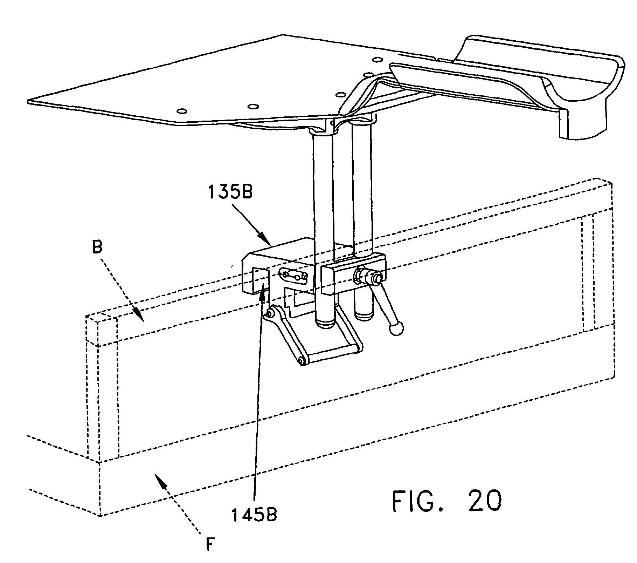

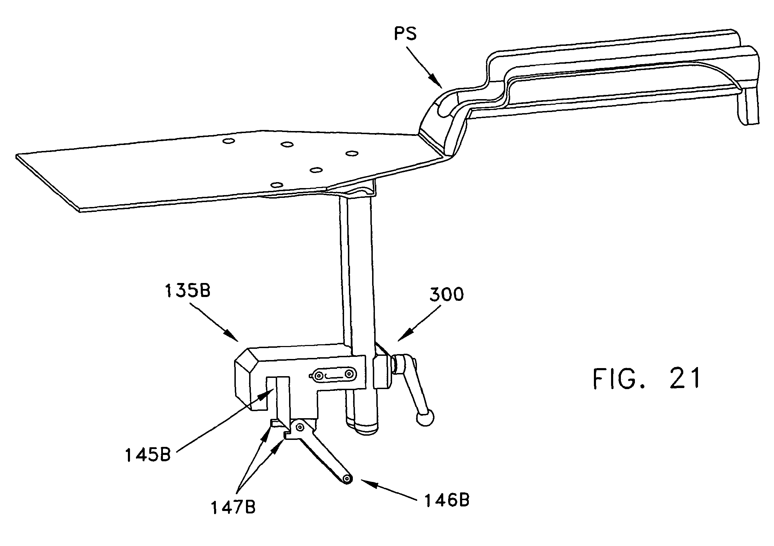

Figs. 20 and 21 show another alternative bed and gurney mount 135B. Bed and gurney mount 135B generally comprises a rectangular bottom slot 145B which receives a rectangular horizontal bar B of frame F. Due to the rectangular profiles of bottom slot 145B and bar B, bed and gurney mount 145B will be rotationally stable relative to horizontal bar B. A lever latch 146B, including fingers 147B, releasably secures bed and gurney mount 135B to the rectangular horizontal bar B. Riser clamp 300 is formed integral with bed and gurney mount 135B.

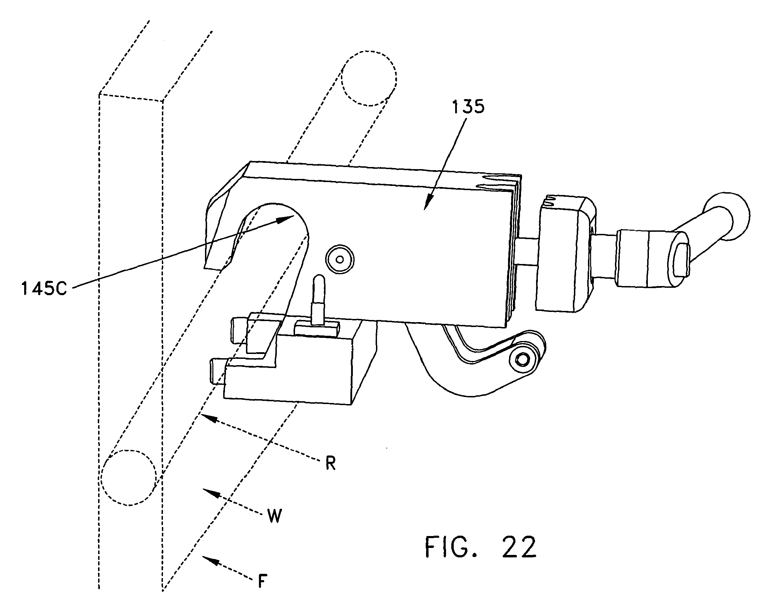

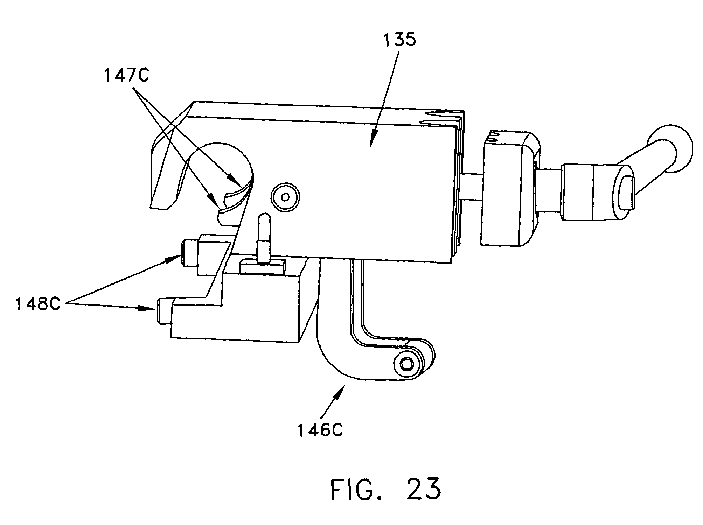

A slightly different arrangement is shown in Figs. 22 and 23. More particularly, in some circumstances, the bed or gurney BG may comprise a frame F which includes a round rod R disposed adjacent to a vertical wall W. In this circumstance, rotational stability must be provided if riser clamp 300 is mounted to the round rod R. Accordingly, and looking now at Figs. 22 and 23, there is shown a bed and gurney mount 135C having a slot 145C formed therein. A lever latch 146C, including fingers 147C, releasably secures bed and gurney mount 135C to the round rod R. A pair of feet 148C bear against wall W of frame F so as to prevent clockwise rotation (when seen from the angle of view of Figs. 22 and 23) of bed and gurney mount 135C relative to round rod R. Riser clamp 300 is formed integral with bed and gurney mount 135C.

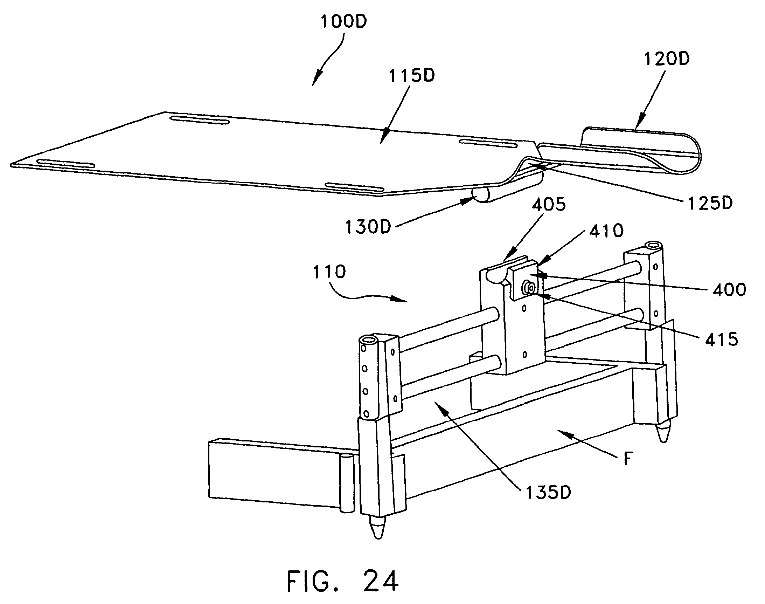

It is also possible to form extender 100 without using risers 130. More particularly, and looking now at Fig. 24, there is shown an extender 100D which generally comprises a shoulder section 115D and a head section 120D, joined by a neck section 125D. A horizontal rod 130D is used to connect extender 100 to adapter 110. In this construction, adapter 110 generally comprises a bed and gurney mount 135D for selective attachment to the frame F of the bed or gurney, and a clamp 400 for clamping horizontal rod 130D in position. Clamp 400 generally comprises a fixed jaw 405 and a movable jaw 410. A screw 415 secures movable jaw to 410 fixed jaw 405.

### Alternative Bed and Gurney Extender Support

It should be appreciated that it is also possible to provide a bed and gurney support element having a construction which is different from bed and gurney support 105 shown in Figs. 4-6.

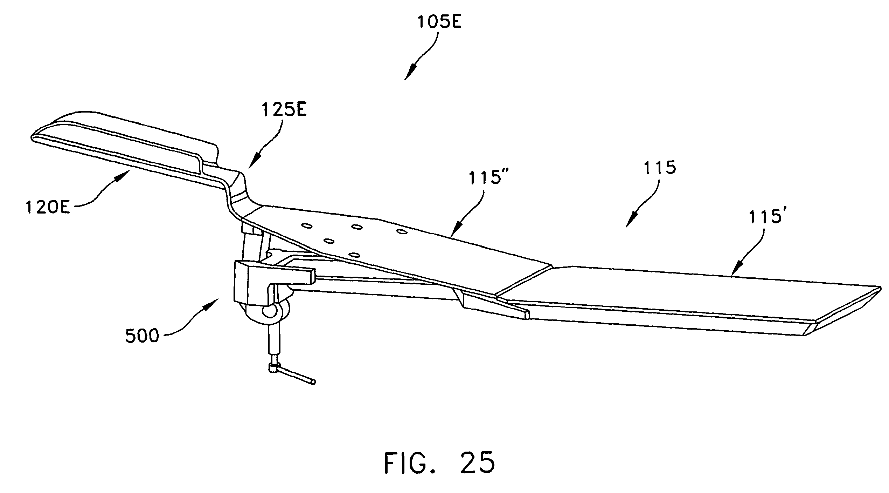

By way of example but not limitation, and looking now at Fig. 25, there is shown a support 105E which permits the patient's head to be aligned with the center opening 20 of CereTom^{™} CT machine 5. To that end, support 105E comprises a hinged shoulder section 115E for disposition under the shoulders of the patient, a head section 120E for supporting of the head of the patient, and a neck section 125E for connecting head section 120E to hinged shoulder section 115E. Hinged shoulder section 115E in turn a first shoulder section 115E' and a second shoulder section 115E". An elevator 500 is provided to raise or lower hinged shoulder section 115E" relative to hinged shoulder section 115E', whereby to permit the patient's head to be aligned with the center opening 20 of CereTom^{™} CT machine 5.

Support 105E can be particularly useful when using an extender such as the extender 100D shown in Fig. 24, inasmuch as extender 100D does not provide vertical adjustment of extender 100D relative to the bed or gurney BG. Elevator 500 of support 105E can provide this vertical adjustment.

### Application To Other Types Of Scanning Systems

It should be appreciated that the present invention is not limited to use with a CereTom^{™} CT machine 5. It may be used with any type of CT machine where the CT machine is capable of moving its scan head relative to a fixed-position patient.

Furthermore, it should be appreciated that the present invention is not limited to use with CT machines. Thus, for example, the present invention may be used in connection with CT machines used for non-medical applications, e.g., with CT machines which are used to scan inanimate objects.

Furthermore, the present invention may be used with non-CT-type scanning systems.

Thus, for example, the present invention may be used with a nuclear medicine diagnostic apparatus such as that disclosed in U.S. Patent No. 6,285,028, issued 09/04/01 to Yamakawa for SEMICONDUCTOR RADIATION DETECTOR AND NUCLEAR MEDICINE DIAGNOSTIC APPARATUS, which patent is hereby incorporated herein by reference, wherein the diagnostic apparatus moves on rails disposed on either side of the patient.

Of course, where the present invention is used in conjunction with scanners using something other than X-rays, it may be necessary to change the composition of head section 120 so that it is rendered transparent in the scanner.

In essence, the present invention has application to any type of mobile imaging system in which the patient (or object) must be scanned on their bed or gurney (or other support).

## Claims

1. A bed and gurney extender (100, 100D) for selective attachment to a bed or gurney (BG) for supporting the head of a patient during scanning, the extender (100, 100D) comprising:
a support (105, 105E) comprising a shoulder section (115, 115D, 115E) for disposition on the bed or gurney (BG) under the shoulders of the patient, and a head section (120, 120D, 120E) for supporting the head of the patient during scanning, wherein at least the head section (120, 120D 120E) is formed out of an x-ray transparent material, the support (105, 105E) comprising a connector element (130, 130D) extending from the underside of the shoulder section of the support (105, 105E), **characterised in that** the connector element is at least one riser (130) or comprises a round horizontal rod (1300); and
an adapter (110) comprising (i) a complementary connector element (140, 300, 400) for securing the connector element (130, 130D) of the support to the adapter (110), wherein the complementary connector element (140, 300, 400) comprises a riser clamp (140, 300) for clamping the at least one riser (130) to the adapter (110) or a clamp (400) for securing the horizontal rod (130D) to the adapter (110), and (ii) a mount (135, 135A, 135B, 135C, 135D) adapted for selectively attaching the adapter (110) to the bed or gurney (BG) so that the extender (100, 100D) is attached to the bed or gurney (BG) for supporting the head of a patient during scanning.

2. An extender according to claim 1 wherein the head section (120, 120D, 120E) is connected to the shoulder section (115, 115D 115E) by a neck section (125).

3. An extender according to claim 2 wherein at least one of the shoulder section (115, 115D, 115E) and neck section (125) are formed out of an X-ray transparent material.

4. An extender according to claim 1 wherein the X-ray transparent material is selected from the group consisting of carbon fiber and plastic.

5. An extender according to claim 2 wherein the head section (120, 120D, 120E) is sized so as to fit within a scan opening (20) of a scanning machine (5).

6. An extender according to claim 5 wherein the shoulder section is (115, 115D, 115E) sized so as to be too large to fit within the scan opening (20) of the scanning machine (5).

7. An extender according to claim 1 wherein the mount (135) comprises at least one receptacle (145) for receiving at least one post (P) provided by the bed or gurney (BG).

8. An extender according to claim 7 wherein the mount (135) comprises two receptacles (145) for receiving two posts (P) provided by the bed or gurney (BG).

9. An extender according to claim 1 wherein the riser clamp (140) comprises a fixed plate (150) and a movable plate (155) which together define at least one variably-sized opening (160).

10. An extender according to claim 9 wherein the at least one variably-sized opening is configured for selectively receiving and securing the at least one riser (130) of the support (105).

11. An extender according to claim 9 wherein the riser clamp (140) is configured to be cam-operated.

12. An extender according to claim 9 wherein the riser clamp (300) is configured to be crank-operated.

13. An extender according to claim 1 wherein the support (105, 105E) further comprises a soft patient support disposed atop the support (105, 105E).

14. An extender according to claim 1 wherein the mount (135A) comprises at least one post (145A) for inserting into at least one hole (H) provided in the bed or gurney (BG).

15. An extender according to claim 1 wherein the mount (135A) comprises two posts (145A) for inserting into two holes (H) provided in the bed or gurney (BG).

16. An extender according to claim 1 wherein the mount (135B, 135C) comprises a horizontally extending slot (145B, 145C) for receiving a portion of the frame (F) of the bed or gurney (BG).

17. An extender according to claim 16 wherein the horizontally extending slot has a rectangular cross-section (145B).

18. An extender according to claim 17 wherein the mount (135B) further comprises movable fingers (147B, 147C) for selectively engaging the portion of the frame (F) of the bed or gurney (BG), whereby to selectively lock the mount (135B) to the frame (F) of the bed or gurney (BG).

19. An extender according to claim 16 wherein the horizontally extending slot (145C) has a round cross-section.

20. An extender according to claim 19 wherein the mount (135C) further comprises movable fingers (147C) for selectively engaging the portion of the frame (F) of the bed or gurney (BG), and further wherein the mount (135C) further comprises (148C) feet for selectively engaging a portion of the frame (F) of the bed or gurney (BG), whereby to selectively lock the mount (135C) to the frame of the bed or gurney (BG).

21. Apparatus for use in scanning a patient on a bed or gurney (BG), comprising:
a bed and gurney extender according to any of the preceeding claims, and
a bed or gurney (BG) **characterized by** an attachment feature, and further wherein the mount (135) is configured to mate with the attachment feature.

22. Apparatus according to claim 21 wherein the apparatus further comprises a mobile scanner (5).

23. Apparatus according to claim 22 wherein the mobile scanner (5) comprises at least one post (210), and further wherein at least a portion of the extender (100) is configured for selected attachment to the at least one post (210) whereby the extender (100) can be selectively mounted to the mobile scanner (5).

## Patentansprüche

1. Erweiterung (100, 100D) für ein Bett oder eine Krankentrage, zur selektiven Befestigung an ein Bett oder an eine Krankentrage (BG), mit Hilfe derer der Kopf des Patienten beim Scannen gestützt wird, wobei die Erweiterung (100, 100D) Folgendes aufweist:
- eine Stütze (105, 105E) mit einem Schulterstück (115, 115D, 115E) zur Anordnung auf dem Bett oder der Krankentrage (BG) unter der Schulter des Patienten und ein Kopfstück (120, 120D, 120E) zur Unterstützung des Kopfs des Patienten beim Scannen, wobei zumindest das Kopfstück (120, 120D, 120E) aus einem röntgenstrahlendurchlässigen Material besteht und wobei die Stütze (105, 105E) ein Verbindungselement (130, 130D) aufweist, das von der Unterseite des Schulterstücks der Stütze (105, 105E) absteht,
**dadurch gekennzeichnet, dass**
das Verbindungselement aus mindestens einem Standrohr (130) besteht oder eine runde, horizontale Stange (130D) aufweist; und
- ein Adapter (110) bestehend aus (i) einem zusätzlichen Verbindungselement (140, 300, 400) zur Befestigung des Verbindungselements (130, 130D) der Stütze an dem Adapter (110), wobei das zusätzliche Verbindungselement (140, 300, 400) ein Spannstück (140, 300) zur Befestigung mindestens eines Standrohrs (130) am Adapter (110) oder ein Spannstück (400) zur Befestigung des horizontalen Stange (130D) am Adapter (110) aufweist, und (ii) ein Lagerelement (135, 135A, 135B, 135C, 135D), das so ausgelegt ist, dass der Adapter (110) entweder am Bett oder an der Krankentrage (BG) befestigt werden kann, so dass die Erweiterung (100, 100D) am Bett oder an der Krankentrage (BG) befestigt ist, um den Kopf des Patienten beim Scannen zu stützen.

2. Erweiterung nach Anspruch 1,
**dadurch gekennzeichnet, dass**
das Kopfstück (120, 120D, 120E) über einen Halsabschnitt (125) mit dem Schulterstück (115, 115D, 115E) verbunden ist.

3. Erweiterung nach Anspruch 2
**dadurch gekennzeichnet, dass**
mindestens eines der Schulterstücke (115, 115D, 115E) und der Halsabschnitt (125) aus einem röntgenstrahlendurchlässigen Material bestehen.

4. Erweiterung nach Anspruch 1
**dadurch gekennzeichnet, dass**
das röntgenstrahlendurchlässige Material aus einem Element der Gruppe bestehend aus Kohlefasern und Kunststoff besteht.

5. Erweiterung nach Anspruch 2,
**dadurch gekennzeichnet, dass**
das Kopfstück (120, 120D, 120E) so dimensioniert ist, dass es in eine Scanöffnung (20) eines Scanners (5) passt.

6. Erweiterung nach Anspruch 5,
**dadurch gekennzeichnet, dass**
Schulterstück (115, 115D, 115E) so dimensioniert ist, dass es zu groß ist, um in eine Scanöffnung (20) eines Scanners (5) zu passen.

7. Erweiterung nach Anspruch 1,
**dadurch gekennzeichnet, dass**
das Lagerelement (135) mindestens eine Aufnahme (145) für mindestens einen Pfosten (P) des Bettes oder der Krankentrage (BG) aufweist.

8. Erweiterung nach Anspruch 7,
**dadurch gekennzeichnet, dass**
das Lagerelement (135) zwei Aufnahmen (145) für zwei Pfosten (P) des Bettes oder der Krankentrage (BG) aufweist.

9. Erweiterung nach Anspruch 1,
**dadurch gekennzeichnet, dass**
das Spannstück (140) eine feste Platte (150) und eine bewegliche Platte (155) aufweist, die zusammen mindestens ein Öffnung mit variablen Abmessungen (160) bestimmen.

10. Erweiterung nach Anspruch 9,
**dadurch gekennzeichnet, dass**
mindestens eine Öffnung mit variablen Abmessungen so ausgelegt ist, dass sie mindestens ein Standrohr (130) der Stütze (105) aufnehmen und sichern kann.

11. Erweiterung nach Anspruch 9,
**dadurch gekennzeichnet, dass**
das Spannstück (140) so ausgelegt ist, dass es sich über Nocken betätigen lässt.

12. Erweiterung nach Anspruch 9,
**dadurch gekennzeichnet, dass**
das Spannstück (300) so ausgelegt ist, dass es sich über eine Kurbel betätigen lässt.

13. Erweiterung nach Anspruch 1,
**dadurch gekennzeichnet, dass**
die Stütze (105, 105E) mit einer weichen Auflage für den Patienten versehen ist, die auf die Stütze (105, 105E) aufgelegt ist.

14. Erweiterung nach Anspruch 1,
**dadurch gekennzeichnet, dass**
das Lagerelement (135A) mindestens einen Pfosten (145A) aufweist, der in mindestens eine Öffnung (H) im Bett oder in der Krankentrage (BG) eingeführt wird.

15. Erweiterung nach Anspruch 1,
**dadurch gekennzeichnet, dass**
das Lagerelement (135A) zwei Pfosten (145A) aufweist, die in zwei Öffnungen (H) im Bett oder in der Krankentrage (BG) eingeführt werden.

16. Erweiterung nach Anspruch 1,
**dadurch gekennzeichnet, dass**
das Lagerelement (135B, 135C) zur Aufnahme eines Teils des Rahmens (F) des Betts oder der Krankentrage (BG) eine horizontal verlaufende Nut (145B, 145C) aufweist.

17. Erweiterung nach Anspruch 16,
**dadurch gekennzeichnet, dass**
die horizontal verlaufende Nut (145B) einen rechteckigen Querschnitt aufweist.

18. Erweiterung nach Anspruch 17,
**dadurch gekennzeichnet, dass**
das Lagerelement (135B) des weiteren bewegliche Ausleger (147B, 147C) aufweist, die in einen Teil des Rahmens (F) des Betts oder der Krankentrage (BG) eingreifen, wodurch das Lagerelement (135B) am Rahmen des Betts oder der Krankentrage (BG) befestigt wird.

19. Erweiterung nach Anspruch 16,
**dadurch gekennzeichnet, dass**
die horizontal verlaufende Nut (145C) einen kreisförmigen Querschnitt aufweist.

20. Erweiterung nach Anspruch 19,
**dadurch gekennzeichnet, dass**
das Lagerelement (135C) des weiteren bewegliche Ausleger (147C) aufweist, die in einen Teil des Rahmens (F) des Betts oder der Krankentrage (BG) eingreifen und wobei das Lagerelement (135C) des weiteren Füße (148C) aufweist, die in einen Teil des Rahmens (F) des Betts oder der Krankentrage (BG) eingreifen, wodurch das Lagerelement (135C) am Rahmen (F) des Betts oder der Krankentrage (BG) befestigt wird.

21. Gerät zur Verwendung beim Scannen eines Patienten auf einem Bett oder auf einer Krankentrage (BG), unit:
- einer Erweiterung für das Bett und für die Krankentrage nach einem der vorgenannten Ansprüche,
- einem Bett oder einer Krankentrage (BG) mit einer Befestigungsvorrichtung, wobei das Lagerelement (135) des weiteren so ausgelegt ist, dass es mit der Befestigungsvorrichtung zusammenwirkt.

22. Gerät nach Anspruch 21,
**dadurch gekennzeichnet, dass**
das Gerät des weiteren einen mobilen Scanner (5) aufweist.

23. Gerät nach Anspruch 22,
**dadurch gekennzeichnet, dass**
der mobile Scanner (5) mindestens einen Pfosten (210) aufweist und wobei mindestens ein Teil der Erweiterung (100) so ausgelegt ist, dass sich diese an mindestens dem einen Pfosten (210) befestigen lässt, wodurch sich die Erweiterung (100) am mobilen Scanner (5) anbringen lässt.

## Revendications

1. Rallonge (100, 100D) pour un lit ou un brancard, pour le montage sélectif à un lit ou à un brancard (BG), servant à l'appui de la tête d'un patient durant le scanning, la rallonge comprenant :
- un support (105, 105E) avec un élément pour les épaules (115, 115D, 115E) qui est placé sur le lit ou sur le brancard en-dessous des épaules du patient, un élément pour la tête (120, 120D, 120E) servant à l'appui de la tête d'un patient durant le scanning, où au moins l'élément de tête 120, 120D, 120E) est réalisé en matériau perméable aux rayons X, le support (105, 105E) possédant un élément de raccordement (130, 130D) saillant du dessous de l'élément pour épaules du support (105, 105E),
**caractérisé en ce que**
l'élément de raccordement consiste au moins d'un tube élévateur (130) ou qu'il comprend une barre horizontale ronde (130D); et
- un adaptateur (110) comprenant (i) un élément de raccordement supplémentaire (140, 300, 400) pour le positionnement de l'élément de raccordement (130, 130D) du support sur l'adaptateur (110), l'élément de raccordement supplémentaire (140, 300, 400) possédant un élément de serrage (140, 300) pour le serrage d'au moins un tube élévateur (130) à l'adaptateur (110), ou un élément de serrage (400) pour le montage de la barre horizontale (130D) sur l'adaptateur (110), et (ii) un élément de support (135, 135A, 135B, 135C, 135D) conçu pour le montage sélectif de l'adaptateur (110) au lit ou au brancard (BG), de sorte que la rallonge (100, 100D) est fixé au lit ou au brancard (BG) pour supporter la tête du patient durant le scanning.

2. Rallonge d'après la revendication 1
**caractérisé en ce que**
l'élément pour la tête (120, 120D, 120E) est raccordé par un col (125) à l'élément pour épaules (115, 115D, 115E).

3. Rallonge d'après la revendication 2
**caractérisé en ce que**
au moins un des éléments pour épaules (115, 115D, 115E) et le col (125) sont réalisés en matériau perméable aux rayons X.

4. Rallonge d'après la revendication 1
**caractérisé en ce que**
le matériau perméable aux rayons X est sélectionné dans un groupe consistant de fibres de carbone et de matières plastiques.

5. Rallonge d'après la revendication 2
**caractérisé en ce que**
l'élément pour la tête (120, 120D, 120E) est dimensionné de sorte à aller dans une ouverture de scanning (20) d'un scanner (5).

6. Rallonge d'après la revendication 5
**caractérisé en ce que**
l'élément pour l'épaules (115, 115D, 115E) est dimensionné de sorte à être trop grand pour aller dans une ouverture de scanning (20) d'un scanner (5).

7. Rallonge d'après la revendication 1
**caractérisé en ce que**
l'élément de support (135) comprend au moins un logement (145) recevant au moins un montant (P) du lit ou du brancard (BG).

8. Rallonge d'après la revendication 7
**caractérisé en ce que**
l'élément de support (135) comprend deux logements (145) recevant deux montants (P) du lit ou du brancard (BG).

9. Rallonge d'après la revendication 1
**caractérisé en ce que**
l'élément de raccordement (140) comprend une plaque fixe (150) et une plaque mobile (155) définissant ensemble au moins une ouverture à grandeur variable (160).

10. Rallonge d'après la revendication 9
**caractérisé en ce que**
au moins une ouverture à grandeur variable est conçue de sorte à recevoir et à fixer au moins un tube élévateur (130) du support (105).

11. Rallonge d'après la revendication 9
**caractérisé en ce que**
l'élément de raccordement (140) est conçu de sorte à être actionné par une came.

12. Rallonge d'après la revendication 9
**caractérisé en ce que**
l'élément de raccordement (300) est conçu de sorte à être actionné par une manivelle.

13. Rallonge d'après la revendication 1
**caractérisé en ce que**
le support (105, 105E) comprend un support souple pour le patient, ce support souple étant placé sur le support (105, 105E).

14. Rallonge d'après la revendication 1
**caractérisé en ce que**
l'élément de support (135A) comprend au moins un montant (145A) qui entre dans au moins une ouverture (H) du lit ou du brancard (BG).

15. Rallonge d'après la revendication 1
**caractérisé en ce que**
l'élément de support (135A) comprend deux montants (145A) qui entrent dans deux ouvertures (H) du lit ou du brancard (BG).

16. Rallonge d'après la revendication 1
**caractérisé en ce que**
l'élément de support (135B, 135C) comprend une gorge horizontale (145B, 145C) recevant une partie du cadre (F) du lit ou du brancard (BG).

17. Rallonge d'après la revendication 16
**caractérisé en ce que**
la gorge horizontale (145B) a une section transversale rectangulaire.

18. Rallonge d'après la revendication 17
**caractérisé en ce que**
l'élément de support (135B) possède de plus des doigts mobiles (147B, 147C) s'engageant sélectivement dans une partie du cadre (F) du lit ou du brancard (BG) afin de fixer l'élément de support (135B) au cadre (F) du lit ou du brancard (BG).

19. Rallonge d'après la revendication 16
**caractérisé en ce que**
la gorge horizontale (145C) a une section transversale ronde.

20. Rallonge d'après la revendication 19
**caractérisé en ce que**
l'élément de support (135C) possède de plus des doigts mobiles (147C) s'engageant sélectivement dans une partie du cadre (F) du lit ou du brancard (BG), et où l'élément de support (135C) possède de plus des pieds (148C) s'engageant sélectivement dans une partie du cadre (F) du lit ou du brancard (BG), afin de fixer l'élément de support (135C) au cadre du lit ou du brancard (BG).

21. Appareil servant à scanner un patient sur un lit ou un brancard (BG), comprenant :
- une rallonge pour un lit ou un brancard d'après une des revendications précédentes
- un lit ou un brancard (BG) équipé d'un dispositif de fixation, et où le support (135) est conçu pour collaborer avec le dispositif de fixation.

22. Appareil d'après la revendication 21
**caractérisé en ce que**
l'appareil comprend de plus un scanner mobile (5).

23. Appareil d'après la revendication 22
**caractérisé en ce que**
le scanner mobile (5) comprend au moins un montant (210), et où au moins une partie de la rallonge (100) est conçue pour le raccordement à au moins un montant (210) afin de pouvoir monter la rallonge (100) au scanner mobile (5).
